# EUROPEAN PATENT SPECIFICATION

(11) **EP 1 575 542 B1**
(45) Date of publication and mention of the grant of the patent: **05.01.2011**
(21) Application number: 03810536.7
(22) Date of filing: 06.11.2003
(51) Int. Cl.: A61Q 9/02, A61Q 17/02, A61K 8/04, A61K 8/41, A61L 9/015

(54) **AEROSOL DELIVERY SYSTEMS**
AEROSOLABGABESYSTEME
SYSTEMES AEROSOLS DE DELIVRANCE

(30) Priority: 06.11.2002 US 288590
(43) Date of publication of application: 21.09.2005
(73) Proprietor: Drug Delivery Solutions Limited, Leatherhead, Surrey KT22 7RY (GB)
(72) Inventor: MERCURIO, Anthony Fred, New Jersey, NJ 07675 (US); WHEELER, Derek Alfred, DISPERSE LIMITED, Guildford, Surrey GU2 6YF (GB)
(74) Representative: Hayes, Adrian Chetwynd
(86) International application number: PCT/GB2003/004806
(87) International publication number: WO 2004/041227

(56) References cited:
- WO-A-2004/002437
- US-A- 4 486 333
- US-A1- 2002 058 055

## Description

The present invention relates to aerosol delivery systems and, in particular, to aerosol delivery systems which are designed to reduce the amount of surfactant included therein.

Aerosol compositions are known in the art which contain oil soluble functional materials such as fragrances, silicones, esters and bio-active materials therein. In order to disperse the oil soluble functional material into the aqueous phase of the aerosol composition there is generally a requirement to include in the composition from two to three times by weight of the functional material of a solvent or surfactant therein.

We have now found that incorporation of the oil soluble functional material into a biliquid foam enables this material to be readily dispersed throughout the aqueous phase of the aerosol composition without the use of excessive amounts of solvents or surfactants, which may affect the material and which may neutralize the effects of any preservatives contained within the aerosol composition.

Accordingly, the present invention provides a pressurized aerosol can comprising an aerosol composition which is prepared from a biliquid foam, an aqueous phase and a propellant, wherein the biliquid foam incorporates an oil soluble functional material therein.

The invention also provides a process for preparing the pressurized aerosol can of the invention which comprises adding the aqueous phase to a vessel, adding the biliquid foam to the vessel and mixing and filling an aerosol can with the addition of a suitable propellant.

Biliquid foams are known in the art and are described in the following literature references by Sebba: "Biliquid foams", J. Colloid and Interface Science, 40 (1972) 468-474; and "The Behaviour of Minute Oil Droplets Encapsulated in a Water Film", Colloid Polymer Sciences, 257 (1979) 392-396.

US Patent No. 4486333 to Sebba describes a particular method for the preparation of biliquid foams by agitating a hydrogen bonded liquid containing a soluble surfactant to produce a gas foam and intermittently adding to the gas foam a non-polar liquid which is immiscible with the hydrogen bonded liquid, the surfactant-containing hydrogen bonded liquid being selected to provide a spreading coefficient equal to or greater than zero.

US 2002/0058055A1 discloses a cosmetic or pharmaceutical system comprising an oil-containing biliquid foam dispersed in a salt-containing aqueous phase. The aqueous phase has a pH of less than about 7 and is gelled by a polymeric sulfonic acid.

The biliquid foam which is incorporated into the aerosol compositions contains at least one oil soluble functional material therein. Examples of the oil soluble functional materials are fragrances, lubricants, vegetable oils, fuels, silicones, esters and bio-active materials.

The biliquid foam which is used in the present invention will preferably comprise from 70 to 95% by weight of the oil phase, which may consist solely of the oil soluble functional material, and 5 to 30% by weight of the continuous phase, which is preferably an aqueous phase. A surfactant to stabilise the biliquid foam may also be included in an amount of from 0.1 to 3%, preferably 0.1 to 1% by weight based on the total weight of the formulation. Suitable surfactants are, for example, sodium lauryl ether sulphates, polyethoxylated castor oil, ethoxylated oleyl alcohols or polyethoxylated hydrogenated castor oils.

propellants which are used in aerosol delivery systems are well known in the art and will preferably be liquified petroleum gas (LPG), which is preferably butane, optionally in admixture with propane. The propellant will generally be present in an amount of from 5 to 40% by weight.

The aerosol compositions which may be used in the present invention will generally include the biliquid foam in an amount of from 0.1 to 10% by weight. The biliquid foam will therefore generally provide the desired oil soluble functional material in an amount of from 0.07 to 9.5% by weight.

The aerosol composition may comprise from 0.01 to 40% by weight of the biliquid foam, from 5 to 40% weight of the propellant and from 20 to 95% by weight of water.

The aqueous phase of the aerosol composition may include therein one or more surfactants or other additives. The surfactant may be chosen to prevent interaction of the propellant with the biliquid foam. Cationic surfactants are preferred, in particular quaternary ammonium compounds or amine oxides. Surfactants may also be chosen to create an affinity with the propellant.

The aerosol compositions which may be used in the present invention may be packaged in cans which are well known in the art and generally are formed of aluminium or lacquered or unlacquered tin plate.

Methods of producing biliquid foams are described in US-A-4486333 involving the preliminary formation of a gas foam in order to provide a sufficiently large surface area on which the biliquid foam can subsequently be formed. It has been found that the prior formation of a gas foam is not required to manufacture a stable biliquid foam, provided that a suitable stirring mechanism is provided in the manufacturing vessel.

Such an apparatus comprises a tank provided with a stirrer in which the stirrer blade breaks the interface between the liquid and air and provides low shear mixing throughout the whole of the volume of the biliquid foam throughout the whole of the production process. A delivery device is provided through which the oil phase (non-polar liquid), which will comprise the internal phase of the dispersion is delivered to the tank. The design of the delivery device is such that the rate of addition of the internal phase fluid can be controlled and varied during the production process. A feature of the production process is that the internal (oil) phase is added to the stirred aqueous phase slowly at first until sufficient droplets have been formed to constitute a large, additional surface area for the more rapid formation of new droplets. At this point, the rate of addition of the oil phase may be increased.

The production process consists of the following steps:
1. The addition of one or more chosen surfactants to one or other or both phases (as previously determined by experiment).
2. The charging of the aqueous phase into the bottom of a process vessel.
3. The incorporation of the stirrer into the vessel so that it stirs the surface of the aqueous phase.
4. Adjustment of the stirrer speed to a previously determined level.
5. The slow addition of the internal phase whilst continuing to stir at the prescribed speed.
6. The speeding up of the rate of addition of the oil phase once a prescribed amount (usually between 5% and 10% of the total amount to be added) has been added.

The stirring rate and the rate of addition of the oil phase are variables, the values of which depend upon the detailed design of the manufacturing plant (in particular, the ratio of tank diameter to impeller diameter), the physico-chemical properties of the oil phase and the nature and concentrations of the chosen surfactants. These can all be pre-determined by laboratory or pilot plant experiment.

It will be understood by those skilled in the art that other manufacturing methods for the biliquid foam may be used, as appropriate.

The preparation of the biliquid foams proceeds independently of the preparation of the final aerosol compositions of the invention. The aerosol compositions may be prepared by adding the aqueous phase, optionally including one or more surfactants therein to a suitable vessel, adding the biliquid foam thereto and mixing. The composition so prepared is then filled into aerosol cans using techniques known in the art. The compositions are then pressurized in the aerosol cans, with the addition of a suitable propellant, using techniques known in the art.

The aerosol composition which may be used in the present invention will generally possess one or more of the following advantages:
- the elimination of the need for the use of large amounts of solvents or surfactants and volatile organic compounds.
- the potential to reduce skin irritation in compositions which are to be applied to the skin,
- the possibility to include in the composition oils which would generally be incompatible with one another;
- the possibility of using lower levels of fragrance components, whilst obtaining the same level of fragrance impact.
- the possibility of using lower levels of preservatives, whilst obtaining the same level of preservation.
- better performing formulations which allow dispensing using less propellant to achieve similar results.

The aerosol compositions which may be used in the invention are preferably used as or in polishes, particularly furniture polishes, air fresheners, fragrances/ moisturisers, sunscreens, shaving preparations or follicle softeners.

The aerosol compositions which may be used in the invention may contain other components (in addition to the biliquid foam, aqueous phase and propellant) depending upon the uses to which they are to be put. Thus, where the aerosol compositions are to be used as polishes, the compositions may additionally contain waxes, for example vegetable waxes (for example, carnauba and candelilla), optionally combined with one or more softeners, fillers and pigments. One or more alcohols or other solvents may also be present.

Where the aerosol compositions are used as furniture polishes, these may additionally contain one or more of silicone, red oil, lemon oil and petroleum solvent; nail polishes generally comprise nitrocellulose, optionally with amyl acetate solvent present.

Where the aerosol compositions are used as air fresheners, a perfumed component (e.g. a free perfume) will generally be present. In addition, such compositions may comprise one or more of the group comprising porosity modifiers, disintegrants, water-swelling agents and colourants. Also present may be inert fillers, hygroscopic agents, binders, coating materials and moisture-providing agents.

Where the compositions are used as repellents, these will generally comprise an active repellent agent such as citronella oil, dimethyl phthalate, n-butylmesityl oxide oxalate and 2-ethyl hexane-1,3-diol. Actidione may be used as an active agent in rodent repellents as may thiuram disulfide, amino complexes with trinitrobenzene and hexachlorophene.

Where the aerosol compositions are used as shaving preparations, appropriate additional components may be as described in Harry's Cosmeticology, 7th Ed., J. B. Wilkinson and R. J. Moore (editors), Chemical Publishers, New York, 1982, pp. 126-189.

Sunscreens will generally comprise either of both a UV-A or UV-B filter. UV-A filters are generally derivates of dibenzoylmethane, particularly avobenzone (4-(1,1-dimethylethyl)-4'-methoxydibenzoylmethane, sold under the brand name PARSOL 1789). Preferably, each of avobenzone, octyl salicylate and oxybenzone is present. Other diabenzoylmethane derivatives known to be UV-A filters are described in US Patent Nos. 4,387,089, 4,489,057 and 4,562,067. UV-B filters are generally paramethoxycinnamic acid esters, such as 2-ethylhexyl paramethoxycinnamate, generally known as octyl methoxy cinnamate or PARSOL MCX, octyl salicylate and oxybenzone.

Follicle softeners generally comprise one or more of the following: surfactants, lubricants, humectants, foaming agents, fragrances, fatty acids and bases.

The present invention will be further described with reference to the following Examples. The Examples describe aerosol compositions suitable for use in the pressurized aerosol can of the present invention

### Example 1

### Preparation of biliquid foam

A biliquid foam was prepared from the following ingredients using the stirring method as described above. The aqueous phase was introduced into a beaker equipped with a stirrer, the diameter of which was approximately 80% of the beaker diameter and the depth sufficient to provide mixing throughout the body of the biliquid foam once the oil addition was complete, to provide low shear mixing. The fragrance and surfactant were slowly added over a period of a few minutes with stirring continuing after completion of the oil addition until the sample became homogeneous.

| **Oil Phase** | % w/w |
|---|---|
| Fragrance | 89.1 |
| Castor oil/Polyoxyethylene glycol | 0.9 |
| (35) adduct (Etocas 35 NF) | |

### Aqueous Phase

| | |
|---|---|
| Demin. water | 9.90 |
| Sodium lauryl ether sulphate | |
| (Standopol) | 0.10 |
| | 100.00 |

### Preparation of Screening Aerosol Composition

An aerosol formulation was prepared from the following ingredients:

| | % w/w |
|---|---|
| Biliquid foam | 0.34 |
| Polyquaternium-11 (Gafquat 755N) | 5.00 |
| Isopentane | 5.00 |
| Water | 89.66 |
| | 100.00 |

The water was mixed with the polyquaternium-11 and the biliquid foam added. The isopentane was then added to the mixture. The mixture demonstrated the suitability of the invention for formulation as an aerosol composition using a suitable propellant to replace the isopentane.

### Example 2

A screening aerosol formulation was prepared from the following ingredients:

| | % w/w |
|---|---|
| Biliquid foam of Example 1 | 0.34 |
| Polyquaternium-11 (Gafquat 755N) | 0.10 |
| Isopentane | 5.00 |
| Water | 94.56 |
| | 100.00 |

The water was mixed with the polyquaternium-11 and the biliquid foam added. The isopentane was then added to the mixture.

### Example 3

A screening aerosol formulation was prepared from the following ingredients:

| | % w/w |
|---|---|
| Biliquid foam of Example 1 | 0.34 |
| Polyquaternium-11 (Gafquat 755N) | 0.05 |
| Isopentane | 5.00 |
| Water | 94.61 |
| | 100.00 |

The water was mixed with the polyquaternium-11 and the biliquid foam added. The isopentane was then added to the mixture.

### Example 4

A screening aerosol formulation was prepared from the following ingredients.

| | % w/w |
|---|---|
| Biliquid foam of Example 1 | 0.34 |
| Polyquaternium-7 (Mackernium 007) | 0.10 |
| Isopentane | 5.00 |
| Water | 94.56 |
| | 100.00 |

The water was mixed with the polyquaternium-11 and the biliquid foam added. The isopentane was then added to the mixture.

### Example 5

A screening aerosol formulation was prepared from the following ingredients:

| | % w/w |
|---|---|
| Biliquid foam of Example 1 | 0.34 |
| Dicetyl Dimonium Chloride | |
| (Proquat 868-P) | 0.10 |
| Isopentane | 5.00 |
| Water | 94.56 |
| | 100.00 |

The water was mixed with the polyquaternium-11 and the biliquid foam added. The isopentane was then added to the mixture.

### Example 6

A screening aerosol formulation was prepared from the following ingredients:

| | % w/w |
|---|---|
| Biliquid foam of Example 1 | 0.34 |
| Amine oxide (AO-455) | 0.10 |
| Isopentane | 5.00 |
| Water | 96.54 |
| | 100.00 |

The water was mixed with the polyquaternium-11 and the biliquid foam added. The isopentane was then added to the mixture.

### Example 7

A screening aerosol formulation was prepared from the following ingredients:

| | % w/w |
|---|---|
| Biliquid foam of Example 1 | 0.34 |
| Amine oxide (AO-455) | 0.05 |
| Isopentane | 5.00 |
| Water | 94.61 |
| | 100.00 |

The water was mixed with the polyquaternium-11 and the biliquid foam added. The isopentane was then added to the mixture.

### Example 8

A screening aerosol formulation was prepared from the following ingredients:

| | % w/w |
|---|---|
| Biliquid foam of Example 1 | 0.34 |
| Vinyl caprolactam/PVP/Dimethyl-aminomethyl methacrylate copolymer | 0.10 |
| Isopentane | 5.00 |
| Water | 94.56 |
| | 100.00 |

The water was mixed with the polyquaternium-11 and the biliquid foam added. The isopentane was then added to the mixture.

### Example 9

A screening aerosol formulation was prepared from the following ingredients:

| | % w/w |
|---|---|
| Biliquid foam | 0.34 |
| Dimethyl lauryl amine oxide | 0.10 |
| Isopentane | 5.00 |
| Water | 94.56 |
| | 100.00 |

The water was mixed with the polyquaternium-11 and the biliquid foam added. The isopentane was then added to the mixture.

### Example 10

A screening aerosol formulation was prepared from the following ingredients:

| | % w/w |
|---|---|
| Biliquid foam | 0.34 |
| Cocamidopropylamine oxide | 0.10 |
| Isopentane | 5.00 |
| Water | 94.56 |
| | 100.00 |

The water was mixed with the polyquaternium-11 and the biliquid foam added. The isopentane was then added to the mixture.

In a similar manner to the preceding examples, further examples of aerosol compositions which may be used in the invention are prepared from the following components:

### Example 11: Furniture Polish

| | % w/w |
|---|---|
| Mineral Oil | 21.02 |
| Deodorized Mineral Spirits | 1.20 |
| Silicone | 4.50 |
| Laureth -4 | 0.27 |
| Sodium Lauryl Ether Sulfate | 0.30 |
| Preservative | 0.05 |
| Water | 71.76 |
| Carbomer | 0.56 |
| Triethanolamine | 0.34 |

### Example 12: Fragrance / Moisturizer

| | % w/w |
|---|---|
| Water | 55.264 |
| Denatured Alcohol | 20.00 |
| Glycerin | 2.00 |
| Polyacrylamide and C13-14 Isoparaffin and Laureth -7 | 0.60 |
| Nylon-12 | 0.50 |
| Titanium Dioxide | 0.40 |
| Glyceryl Trioctanoate | 5.00 |
| Isododecane | 4.00 |
| Silicone | 6.00 |
| Fragrance | 6.00 |
| PEG 25 Hydrogenated Castor Oil | 0.106 |
| PEG 25 Castor Oil | 0.106 |
| Sodium Lauryl Ether Sulfate | 0.024 |

### Example 13: Air Freshener

| | % w/w |
|---|---|
| Water | 97.98 |
| Carbomer | 0.60 |
| Triethanolamine | 0.30 |
| Preservative | 0.10 |
| Fragrance | 0.45 |
| Laureth - 4 | 0.27 |
| Sodium Lauryl Ether Sulfate | 0.30 |

### Example 14: Sunscreen

| | % w/w |
|---|---|
| Avobenzone | 3.00 |
| Octyl Salicylate | 5.00 |
| Oxybenzone | 4.00 |
| Diethylhexyl 2.6-Naphthalate | 5.00 |
| Sunflower Oil | 2.00 |
| Cyclopentasiloxane | 2.00 |
| PEG 35 Castor Oil | 0.21 |
| Sodium Lauryl Ether Sulfate | 0.24 |
| Water | 73.00 |
| Carbomer | 0.25 |
| Triethanolamine | 0.10 |
| Propylene Glycol | 5.00 |
| Preservative | 0.20 |

## Claims

1. A pressurized aerosol can comprising an aerosol composition comprising a biliquid foam, an aqueous phase and a propellant, wherein the biliquid foam incorporates an oil soluble functional material therein.

2. The pressurized aerosol can as claimed in claim 1 wherein the oil soluble functional material is a fragrance, lubricant, vegetable oil, fuel, silicone, ester or a bioactive material.

3. The pressurized aerosol can as claimed in any one of the preceding claims wherein the propellant is liquefied petroleum gas.

4. The pressurized aerosol can as claimed in any one of the preceding claims wherein the aqueous phase includes therein one or more surfactants, or additives

5. The pressurized aerosol can as claimed in claim 4 wherein the surfactant is a cationic surfactant.

6. The pressurized aerosol can as claimed in claim 5 wherein the cationic surfactant is a quaternary ammonium compound or an amine oxide.

7. The pressurized aerosol can as claimed in any one of the preceding claims wherein the aerosol composition is a polish, air freshener, repellent, pre- or post shave preparation, shaving preparation or follicle softener.

8. The pressurized aerosol can as claimed in claim 1 wherein the aerosol composition comprises from 0.1 to 10% by weight of biliquid foam.

9. The pressurized aerosol can as claimed in claim 1 wherein the aerosol composition comprises from 5 to 40% by weight of propellant.

10. A process for preparing the pressurized aerosol can as defined in any one of claims 1 to 7 which comprises adding the aqueous phase to a vessel, adding the biliquid foam to the vessel and mixing, and filling the aerosol can with the addition of a suitable propellant.

## Patentansprüche

1. Unter Druck stehender Aerosolbehälter, der eine Aerosolzusammensetzung enthält, die einen Schaum aus zwei Flüssigkeiten, eine wässrige Phase und ein Treibmittel umfasst, wobei der Schaum aus zwei Flüssigkeiten ein öllösliches funktionelles Material einschließt.

2. Unter Druck stehender Aerosolbehälter nach Anspruch 1, wobei das öllösliche funktionelle Material ein Parfüm, ein Gleitmittel, ein pflanzliches Öl, ein Treibstoff, ein Silicon, ein Ester oder ein bioaktives Material ist.

3. Unter Druck stehender Aerosolbehälter nach einem der vorhergehenden Ansprüche, wobei es sich bei dem Treibmittel um verflüssigtes Erdgas handelt.

4. Unter Druck stehender Aerosolbehälter nach einem der vorhergehenden Ansprüche, wobei die wässrige Phase einen oder mehrere grenzflächenaktive Stoffe oder Additive einschließt.

5. Unter Druck stehender Aerosolbehälter nach Anspruch 4, wobei der grenzflächenaktive Stoff ein kationischer grenzflächenaktiver Stoff ist.

6. Unter Druck stehender Aerosolbehälter nach Anspruch 5, wobei der kationische grenzflächenaktive Stoff eine quartäre Ammoniumverbindung oder ein Aminoxid ist.

7. Unter Druck stehender Aerosolbehälter nach einem der vorhergehenden Ansprüche, wobei die Aerosolzusammensetzung eine Politur, ein Luftverbesserer, ein Repellent, eine vor oder nach der Rasur aufzutragende Zubereitung, eine Rasierzubereitung oder ein Haarkonditioniermittel ist.

8. Unter Druck stehender Aerosolbehälter nach Anspruch 1, wobei die Aerosolzusammensetzung den Schaum aus zwei Flüssigkeiten in einem Anteil von 0,1 bis 10 Gew.-% enthält.

9. Unter Druck stehender Aerosolbehälter nach Anspruch 1, wobei die Aerosolzusammensetzung 5 bis 40 Gew.-% Treibmittel enthält.

10. Verfahren zur Herstellung des unter Druck stehenden Aerosolbehälters, der wie in einem der Ansprüche 1 bis 7 definiert ist, das das Hineingeben der wässrigen Phase in ein Gefäß, das Hineingeben des Schaums aus zwei Flüssigkeiten in das Gefäß und das Vermischen sowie das Auffüllen des Aerosolbehälters durch Zugabe eines geeigneten Treibmittels umfasst.

## Revendications

1. Boîte d'aérosol sous pression comprenant une composition d'aérosol comprenant une mousse biliquide, une phase aqueuse et un propulseur, où la mousse biliquide incorpore un matériau fonctionnel soluble dans l'huile dans celle-ci.

2. Boîte d'aérosol sous pression selon la revendication 1, où le matériau fonctionnel soluble dans l'huile est une fragrance, un lubrifiant, une huile végétale, un combustible, un silicone, ester ou un matériau bioactif.

3. Boîte d'aérosol sous pression selon l'une quelconque des revendications précédentes, où le propulseur est du gaz de pétrole liquéfié.

4. Boîte d'aérosol sous pression selon l'une quelconque des revendications précédentes, où la phase aqueuse comporte un ou plusieurs agents de surface ou additifs.

5. Boîte d'aérosol sous pression selon la revendication 4, où l'agent de surface est un agent de surface cationique.

6. Boîte d'aérosol sous pression selon la revendication 5, où l'agent de surface cationique est un composé d'ammonium quaternaire ou un oxyde d'amine.

7. Boîte d'aérosol sous pression selon l'une quelconque des revendications précédentes, où la composition d'aérosol est une préparation de polissage, de rafraîchissement d'air, répulsive, de pré ou post-rasage, une préparation de rasage ou adoucisseur de follicules.

8. Boîte d'aérosol sous pression selon la revendication 1, où la composition d'aérosol comprend de 0,1 à 10% en poids de mousse biliquide.

9. Boîte d'aérosol sous pression selon la revendication 1, où la composition d'aérosol comprend de 5 à 40% en poids de propulseur.

10. Procédé de préparation de la boîte d'aérosol sous pression telle que définie dans l'une quelconque des revendications 1 à 7, qui comprend l'addition de la phase aqueuse à un récipient, l'ajout de la mousse biliquide dans le récipient et le mélange, et le remplissage de la boîte d'aérosol avec l'addition d'un propulseur approprié.
